(19) Europäisches Patentamt
European Patent Office
Office européen des brevets

(11) **EP 4 740 852 A1**

(12) **EUROPEAN PATENT APPLICATION**

(43) Date of publication:
**13.05.2026 Bulletin 2026/20**

(21) Application number: **24211955.0**

(22) Date of filing: **11.11.2024**

(51) International Patent Classification (IPC):
***A61B 5/0225*** *(2006.01)*

(52) Cooperative Patent Classification (CPC):
**A61B 5/0225**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventor: **DUINEVELD, Paulus Cornelis**
**Eindhoven (NL)**

(74) Representative: **Philips Intellectual Property & Standards**
**High Tech Campus 52**
**5656 AG Eindhoven (NL)**

(54) **PUMP TO ACTUATOR FLOW RATE**

(57) Proposed concepts thus aim to provide schemes, solutions, concepts, designs, methods and systems pertaining to determining a flow rate from a pump to an actuator of a blood pressure measurement system. In particular, embodiments aim to provide a method determining a flow rate from a pump to an actuator of a blood pressure measurement system. This can be achieved by using a pump operating parameter value which describes an operation of the motor of the pump and a pressure value which is responsive to a pressure inside the actuator.

100

110

Determining a flow rate

**FIG. 1**

Processed by Luminess, 75001 PARIS (FR)

**Description**

FIELD OF THE INVENTION

This invention relates to the field of determining a flow rate from a pump to an actuator.

BACKGROUND OF THE INVENTION

Non-invasive blood pressure measurement technology is known in the art and has the potential to measure with a similar accuracy to more complicated invasive techniques, such as with a catheter.

For example, a non-invasive oscillometric blood pressure system measures blood pressure without penetrating the skin. It works by inflating a cuff around a subject's arm, which temporarily stops blood flow in the artery. As the cuff deflates, the system detects oscillations in the arterial wall caused by blood flow, and these oscillations can be analyzed to determine systolic and diastolic blood pressure values.

Some non-invasive blood pressure measurement systems involve a shell cuff actuator. A shell cuff actuator includes an inflatable cuff which can wrap around, for example, the upper arm, connected to a small pump and pressure sensor. When activated, the actuator inflates the cuff to thus compress the brachial artery, and during inflation or deflation, the hemodynamic parameters are typically measured. The actuator's precise control over inflation and deflation, combined with sensitive pressure detection, allows for accurate and automated blood pressure readings.

To this end, it is useful to know the exact flow rate from a pump to an actuator of a (non-invasive) blood pressure measurement system.

SUMMARY OF THE INVENTION

The invention is defined by the independent claims. The dependent claims define advantageous embodiments.

According to examples in accordance with an aspect of the invention, there is provided a computer-implemented method for determining a flow rate from a pump to an actuator of a blood pressure measurement system.

The method comprises: determining a flow rate from the pump to the actuator based on a pump operating parameter value, describing operation of a motor of the pump, and a pressure value corresponding to a pressure inside the actuator.

Proposed concepts thus aim to provide schemes, solutions, concepts, designs, methods and systems pertaining to determining a flow rate from a pump to an actuator of a blood pressure measurement system. In particular, embodiments aim to provide a method for determining a flow rate from a pump to an actuator of a blood pressure measurement system by using a pump operating parameter value which describes an operation of the motor of the pump and a pressure value which is responsive to a pressure inside the actuator.

In other words, it is proposed that by using these two parameters, the flow rate from the pump to the actuator can be determined. This leverages the fact that the operation of individual pumps is very reproducible. The flow rate can thus be provided using parameters readily available to a standard blood pressure measurement system, e.g., without the need of any extra sensors.

By accurately determining the flow rate delivered by the pump to the actuator it is therefore possible to detect potential issues with the measurement system and to improve control of the system to ensure, for example, a constant actuator pressure slope.

Ultimately, an improved method for determining a flow rate from a pump to an actuator of a blood pressure measurement system is provided.

In some embodiments, the pump operating parameter value comprises at least one of: pulse width modulation percentage of the motor of the pump; and revolutions per minute of the motor of the pump. Both of these pump operating parameter values may be highly effective and efficient parameters to use to help determine the flow rate from the pump to the actuator.

In some embodiments, determining the flow rate from the pump to the actuator is further based on at least one of: voltage supplied to the motor of the pump; pump type; pump state of the pump; ambient pressure; altitude of the actuator; and ambient temperature. These may all be useful further parameters to help make the determined flow rate more accurate.

In some embodiments, the method further comprises: detecting a measurement issue based on the determined flow rate. Based on the determined flow rate, it may be possible to detect a wide range of issues with the blood pressure measurement system.

In some embodiments, the measurement issue comprises at least one of: a partial blockage of a regulating orifice; a full blockage of a regulating orifice; actuator leakage; a partial blockage of a tube coupling the actuator to a measuring device; and a full blockage of a tube coupling the actuator to a measuring device. These may be useful issues to detect as then said issues can be addressed to improve the reliability of the blood pressure measurement system.

In some embodiments, detecting the measurement issue is further based on a subject parameter. This may ensure a more accurate detection of measurement issues.

In some embodiments, a subject parameter comprises at least one of: BMI; weight; height; age; gender; and arm circumference. Each of these may be useful subject parameters to facilitate more accurate detection of measurement issues.

In some embodiments, the method further comprises generating a control signal based on the determined flow rate and the detected measurement issue. This may facilitate automated compensation for the detected measurement issue.

In some embodiments, the control signal comprises a control instruction for modifying an operating parameter of the pump. This may be an effective way to compensate for the detected measurement issue.

In some embodiments, the operating parameter comprises at least one of: pulse width modulation percentage of the motor of the pump; revolutions per minute of the motor of the pump; voltage supplied to the motor of the pump; and user feedback. These operating parameters may all facilitate remedying of or compensation for the determined measurement issue.

In some embodiments, the method further comprises: determining a first volume of fluid supplied to the actuator based on the determined flow rate; determining a second flow rate from the pump to the actuator based on a second pump operating parameter value and a second pressure value, wherein the second pump operating parameter value and the second pressure value describe a different time point to the pump operating parameter value and the pressure value; determining a second volume of fluid supplied to the actuator based on the determined second flow rate; and determining a total volume of fluid supplied to the actuator as a function of pressure value based on the determined first and second volumes of fluid and the first and second pressure values. The total volume of fluid supplied to the actuator as a function of pressure value may be a particularly useful function for assessing operation of the blood pressure measurement system.

In some embodiments, the blood pressure measurement system comprises a regulating orifice, and the method further comprises: predicting an orifice flow rate through the regulating orifice; and adjusting the determined flow rate and/or the determined total volume of fluid as a function of pressure value based on the predicted orifice flow rate. This may allow the flow rate and/or total volume of fluid as a function of pressure value to be made more accurate.

In some embodiments, the method further comprises determining an actuator compliance value based on the determined total volume of fluid as a function of pressure value and the first and second pressure values. The actuator compliance value may be a useful parameter to derive for assessment of the operation of the blood pressure measurement system.

In some embodiments, the blood pressure measurement system is a non-invasive blood pressure measurement system. The present invention may be of particular benefit when using non-invasive blood pressure measurement systems.

In some embodiments, the actuator comprises a shell cuff actuator. The present invention may be of particular benefit when using a shell cuff actuator.

According to another aspect of the invention, there is provided a computer program comprising code for implementing the method of any herein disclosed method when said program is run on a processor.

According to another aspect of the invention, there is provided a blood pressure measurement system. The system comprises a pump; an actuator; and a processor configured to: determine a flow rate from the pump to the actuator based on a pump operating parameter value, describing operation of a motor of the pump, and a pressure value corresponding to a pressure inside the actuator.

Thus, there may be proposed concepts for determining a flow rate from a pump to an actuator of a blood pressure measurement system, and this may be done based on a pump operating parameter value and a pressure value corresponding to a pressure inside the actuator.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a simplified flow diagram of a method for determining a flow rate from a pump to an actuator of a blood pressure measurement system according to a proposed embodiment;
Figs. 2A-E are diagrams illustrating experimental results of the scaling of various parameters of an average Q-H curve at reference conditions with a PWM% signal;
Fig. 3 is a diagram illustrating the fitting of reference flow rate as a function of pressure difference for different PWM% with experimental measurements;

Fig. 4 is a diagram illustrating the experimental results of the effect of voltage on a Q-H curve;

Fig. 5 is a diagram illustrating the experimental results of the correlation of PWM% with RPM and pump state;

Fig. 6 is a flow diagram of a method for determining a flow rate from a pump to an actuator of a blood pressure measurement system according to a proposed embodiment;

Fig. 7 is a flow diagram of a method for determining a flow rate from a pump to an actuator of a blood pressure measurement system according to a proposed embodiment;

Fig. 8 is a simplified block diagram of a system for determining a flow rate from a pump to an actuator of a blood pressure measurement system according to a proposed embodiment; and

Fig. 9 illustrates an example of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to determining a flow rate from a pump to an actuator of a blood pressure measurement system. According to proposed concepts, a number of possible solutions may be implemented separately or jointly. That is, although these possible solutions may be described below separately, two or more of these possible solutions may be implemented in one combination or another.

Embodiments of the invention aim to provide a method for determining a flow rate from a pump to an actuator of a blood pressure measurement system. This can be achieved by using a pump operating parameter value which describes an operation of the motor of the pump and a pressure value which is responsive to a pressure inside the actuator.

In other words, it is proposed that by using these two parameters, the flow rate from the pump to the actuator can be determined. This leverages the fact that the operation of individual pumps is very reproducible. The flow rate can thus be provided using parameters readily available to a standard blood pressure measurement system, e.g., without the need of any extra sensors.

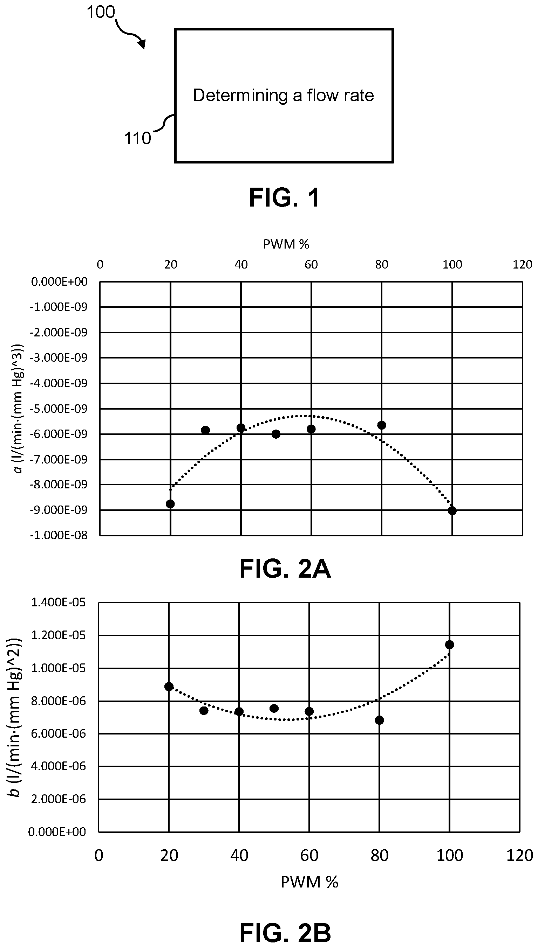

Referring now to Fig. 1, there is depicted a simplified flow diagram of a computer-implemented method 100 for determining a flow rate from a pump to an actuator of a blood pressure measurement system according to a proposed embodiment.

The method 100 comprises step 110 of determining a flow rate from the pump to the actuator. This is based on a pump operating value, which describes operation of a motor of the pump, and a pressure value corresponding to a pressure inside the actuator.

A flow rate can be understood as a measure of the volume of fluid (i.e., gas or liquid, typically air in blood pressure measurement systems) moving from the pump to the actuator per unit of time.

A pump operating value describing (i.e., corresponding to or responsive to) operation of a motor of the pump can be understood as a parameter that reflects the motor's performance such as speed, power, or torque during operation. For example, in this embodiment, the pump operating parameter value specifically comprises at least one of: pulse width modulation percentage of the motor of the pump; and revolutions per minute of the motor of the pump. Both of these pump operating parameter values are highly effective and efficient parameters to use to help determine the flow rate from the pump to the actuator. To be clear, Pulse Width Modulation (PWM) percentage for a motor refers to the control of motor speed by adjusting the proportion of time the motor receives full voltage within each cycle. A higher PWM percentage means the motor is powered for a greater portion of the cycle, leading to higher speed, while a lower percentage reduces the power duration and thus decreases the speed.

In other embodiments, as would be understood by the skilled person, the pump operating value can comprise any other suitable value, such as, for example, motor current or motor torque.

A pressure value corresponding to a pressure inside the actuator can also be understood as a pressure value responsive to a pressure inside the actuator. Responsive to / corresponding to the pressure inside the actuator means that the actual pressure inside the actuator can be derived from the pressure value, or that that the pressure value can be derived from the actual pressure inside the actuator. In other words, the pressure value can be an absolute measurement of the pressure inside the actuator or it can be a proxy for the pressure inside the actuator which varies in accordance with the pressure inside the actuator (for example, a pressure measurement between the pump and the actuator which will be slightly higher than the actual pressure inside the actuator). It should be noted that the pressure value can also be referred to as the gauge pressure, i.e., the pressure difference inside the actuator with respect to the ambient (surrounding) pressure.

To be clear, pressure inside the actuator refers to the force exerted by the fluid (usually air) within the actuator's chamber, e.g., a cuff. This pressure drives the actuator's movement, for example, inflating the cuff so that it tightens around the arm of a subject. Preferably, this pressure can be measured with a pressure sensor which is already present in the blood pressure measurement system (as it is typical for a blood pressure measurement system to include a pressure sensor). For example, a pressure sensor is used in a standard non-invasive oscillometric measure sensor (e.g., for measurements). In another example, a pressure sensor is used in a shell cuff blood pressure measurement system to ensure that the inflation rate of the actuator pressure is as constant as possible.

In this embodiment, the blood pressure measurement system comprises a non-invasive blood pressure measurement system. The present invention is of particular benefit when using non-invasive blood pressure measurement systems. In some embodiments, the blood pressure measurement system can comprise a blood pressure monitoring system (i.e., extended/continuous measurement). For example, in some embodiments, the blood pressure measurement system can comprise a blood pressure oscillometric measurement system.

In an embodiment, the actuator comprises a shell cuff actuator. The present invention is of particular benefit when using a shell cuff actuator. In other embodiments, the actuator may comprise any suitable (non-invasive) actuator (for use in a blood pressure measurement system) such as, for example, a bladder cuff actuator, a wrist cuff actuator, or a finger cuff actuator.

Furthermore, determining the flow rate (in step 110) can be further based on at least one of: voltage supplied to the motor of the pump; pump type; pump state of the pump; ambient pressure; altitude of the actuator; and ambient temperature. These are all be useful further parameters to help make the determined flow rate more accurate. For example, the pump type can comprise at least one of: a centrifugal pump; a positive displacement pump; a diaphragm pump; or any suitable pump type, as the skilled person would appreciate. Pump state refers to the operational mode of the pump at the time point of the pump operating value and pressure value, for example, whether it is running, idling, or malfunctioning. Ambient pressure can be understood as the local atmospheric/environmental/absolute pressure directly around the blood pressure measurement system. Altitude of the actuator can be understood as the altitude of the actuator (and thus the rest of the blood pressure measurement system) relative to sea level - this is essentially a proxy for ambient pressure. The ambient temperature can be understood as the local atmospheric/environmental/absolute temperature directly around the blood pressure measurement system. The skilled person would appreciate that these further parameters are not essential to the working of the invention but merely provide enhanced accuracy of determining the flow rate.

In some embodiments, determining the flow rate in step 110 comprises using a cubic or quadratic formula, however, the skilled person would appreciate that in other embodiments, any suitable formula can be used.

It should be noted that in some embodiments, the method 100 can further comprise actually obtaining the pump operating parameter value and the pressure value. For example, these can be obtained by directly measuring them or by receiving the values from an external system/device. The skilled person would appreciate the many different ways in which these values could be obtained.

In other words, the core concept of the present invention is the use of PWM% information combined with the actuator pressure to calculate the flow rate between a pump and an actuator of a blood pressure measurement system (which may vary depending on the pump type used). Alternatively or in combination, RPM data of the pump can be used instead of (or in addition to) PWM% information. Historical RPM data could also be included in the calculation. In this way, the flow rate can be determined without extra/external information - this is possible because individual pumps are very reproducible/consistent.

The flow rate is influenced by the absolute (ambient) pressure, and so preferably the absolute (ambient) pressure is known, or the altitude where the blood pressure measurement system is being used in order to make the flow rate more accurate.

Furthermore, the pump performance is also influenced by the voltage that is applied to the pump. For a system connected to a power supply, the voltage will be reasonably consistent, however, for a battery driven system, the voltage may change and so it is useful to measure. Voltage data can be used to help calculate a more accurate flow rate.

The flow rate is also influenced by temperature. With information of the local temperature at the position of, for example a pressure sensor of the blood pressure measurement system (which is typically already available), the flow rate can be made more accurate.

In a more specific explanation of the invention, the pumps that are used in a typical blood pressure measurement system are very repeatable, i.e., the spread between pumps is very small. For example, in an experiment using several commonly-used pumps, the error between the worst-case pump and the average was less than 10% up to 450 mm Hg.

The reference flow rate of a pump can be written in a general cubic formula:

$$Q_{ref} = a(p_a, PWM\%, \Delta V, T)\Delta p_{act}^3 + b(p_a, PWM\%, \Delta V, T)\Delta p_{act}^2 +$$

$$c(p_a, PWM\%, \Delta V, T)\Delta p_{act} + Q_{max}(p_a, PWM\%, \Delta V, T) \tag{1}$$

Or a quadratic formula:

$$Q_{ref} = a(p_a, PWM\%, \Delta V, T)\Delta p_{act}^2 + b(p_a, PWM\%, \Delta V, T)\Delta p_{act} + Q_{max}(p_a, PWM\%, \Delta V, T)$$

$$\tag{2}$$

In principle, a linear description is also possible, however, this would be substantially less accurate than a cubic or quadratic expression.

In formulas (1) and (2), each parameter *a, b,* c and $Q_{max}$ is a function of the ambient pressure $p_a$, the voltage applied to the pump, $\Delta V$, the temperature $T$ and the PWM% signal/value.

It is noted that the present disclosure describes that the flow rate can be determined based on only a PWM% value and an actuator pressure value, however, formulas (1) and (2) also include ambient pressure, temperature, and voltage applied to the pump. These additional parameters are not essential to the functioning of the invention but merely provide additional accuracy. This is because in many circumstances, estimates of the additional parameters can be used (i.e., not an actual value, but rather a constant in its place) without a substantial reduction in accuracy.

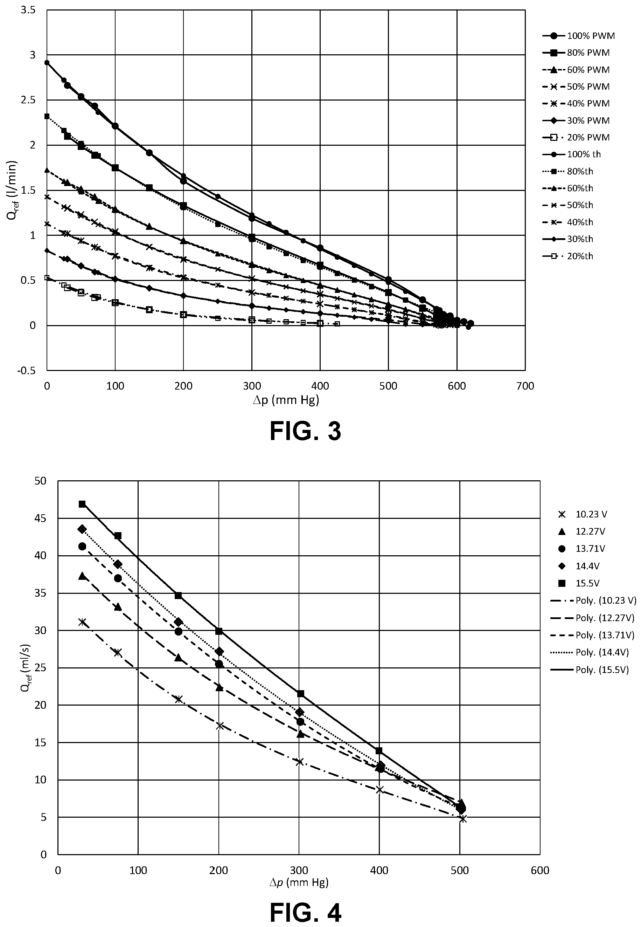

For example, when a pump is connected to mains power, the applied voltage will typically be in between 13.6 and 14.8V. At this voltage range, there is not much variation in pump performance (as can be seen in Fig. 4). Hence, the use of a single, average (e.g., predicted constant) voltage can be used to obtain an accurate flow rate determination instead of an actual voltage measurement/value. In other words, a voltage measurement/value is not essential for the determination of the flow rate, but rather a constant can be used in its place and will be suitable for the majority of circumstances. However, if the pump is connected to batteries as its power source, the voltage variation can be much larger, with the voltage potentially dropping to substantially lower values - in these cases, an actual voltage measurement/value is required for the flow rate determination to remain accurate enough to be useful.

Considering the effect of ambient pressure, as described, the maximum pressure and the maximum reference flow rate both scale linearly with ambient/absolute pressure. Almost 90% of the world's population live below 1000m altitude. At 1000m altitude, the ambient pressure is, on average, 0.89 bar, while at sea level it is 1.0 bar. An ambient pressure constant of 0.95 bar can therefore be used (in the vast majority of cases) in place of an actual ambient pressure measurement/value, thereby allowing an accurate flow rate determination to be obtained without needing an actual ambient pressure measurement/value. With the constant of 0.95 bar, the error will be, at most, 5% for 90% of the population.

Furthermore, there is not much variation in pump performance due to temperature (in the ranges in which the pump will typically be used). Again, a single, average (e.g., predicted constant) temperature can be used to obtain an accurate flow rate determination instead of an actual temperature measurement/value, preferably in a range of 19-25 °C, and more preferably a temperature of 20 or 21 °C. In other words, a temperature measurement/value is not essential for the determination of the flow rate, but rather a constant can be used in its place and will be suitable for the majority of circumstances.

For a pump, an average Q-H (reference flow rate versus pressure difference generated by the pump) curve can be obtained by measuring several pumps and taking the average.

For example, parameters *a, b,* c and $Q_{max}$ can be described at a reference $p_a$ (e.g., 1 bar), reference temperature (21 °C) and reference voltage (14.4V). These relations can be obtained, for example, via experimental measurements of several different pumps from which the average can be obtained. For example, during testing, this was done for seven different PWM% for four different pumps (of the same type). From these results, the scaling of the four parameters of the average pump curve with PWM% could be found. The skilled person would readily understand how this could be done for any type of pump they wish to use, and examples are shown in Figs. 2A-E, with the points representing the actual results and the dotted line representing a fitted curve.

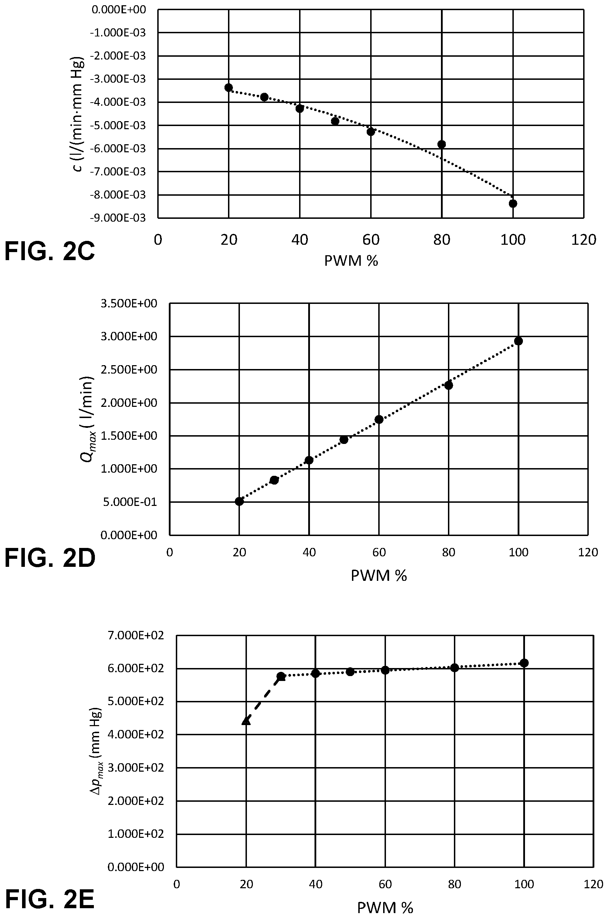

Fig. 2A illustrates experimental results for the scaling of parameter *a* of an average Q-H curve at reference conditions with a PWM% signal. Fig. 2B illustrates experimental results for the scaling of parameter *b* of an average Q-H curve at reference conditions with a PWM% signal. Fig. 2C illustrates experimental results for the scaling of parameter *c* of an average Q-H curve at reference conditions with a PWM% signal. Fig. 2D illustrates experimental results for the scaling of parameter $Q_{max}$ of an average Q-H curve at reference conditions with a PWM% signal. Fig. 2E illustrates experimental results for the scaling of parameter $\Delta p_{max}$ of an average Q-H curve at reference conditions with a PWM% signal.

As seen in Fig. 2E, $\Delta p_{max}$ can be plotted as a function of PWM%, and in this case, it appears to be better to use two linear functions in PWM% to describe $\Delta p_{max}$. From theory of an ideal pump (with no friction) one would expect that by changing the PWM%, $Q_{max}$ of the pump would scale linearly with PWM% (as only the number of pumping cycles of the pump is dropping), while one would expect that $\Delta p_{max}$ of the pump would remain constant, as the voltage per pump stroke remains the same. From Figs. 2D and 2E, it can be seen that this scaling holds well for $Q_{max}$ even up to the lowest PWM% while for $\Delta p_{max}$, it holds well up to around 30% PWM%.

Based on the linear scaling of $Q_{max}$ with PWM% and no effect on $\Delta p_{max}$, one would expect that *a* and *b* hardly change with PWM%, while *c* will scale linearly with PWM%. From experiments, this has proved to approximately be the case. Only fitting results close to $\Delta p_{max}$ show some deviation. This can be clearly observed in Fig. 3, which is a diagram illustrating the fitting of reference flow rate as a function of pressure difference for different PWM% compared with experimental measurements.

In an embodiment, an improvement of fitting results close to $\Delta p_{max}$ is possible (it is noted, however, that in practice it will be quite rare to achieve $\Delta p_{max}$). Close to the experimentally determined $\Delta p_{max}$, the Q-H curve can be described as a linear function:

$$Q_{ref} = K \cdot \left(\Delta p_{max,exp} - \Delta p\right) \tag{3}$$

Where K can be found from equating the derivative of equations (1) and (3) with respect to $\Delta p$:

$$K = -3a\Delta p_{max}^2 - 2b\Delta p_{max} - c \tag{4}$$

Where, for simplicity, an approximation of $\Delta p_{max}$ from the theoretical curve is used, given by:

$$\Delta p_{max} = \frac{-b}{3a} - \frac{2^{\frac{1}{3}}(3ac-b^2)}{3a\left(-2b^3+9abc-27a^2d+\sqrt{4(-b^2+3ac)^3+(-2b^3+9abc-27a^2d)^2}\right)^{\frac{1}{3}}} \tag{5}$$

$$+ \frac{\left(-2b^3 + 9abc - 27a^2d + \sqrt{4(-b^2 + 3ac)^3 + (-2b^3 + 9abc - 27a^2d)^2}\right)^{\frac{1}{3}}}{3a \cdot 2^{\frac{1}{3}}}$$

Equation (3) can then be used for high $\Delta p$ and equation (1) for low $\Delta p$. The point where one should change from one solution to the other (i.e., the point defining the transition between "high" and "low" $\Delta p$) can be found by minimizing the distance between the two curves at the point of change, as the skilled person would appreciate.

In principle, the scaling of pressure and flow rate with $p_a$ and $T$ can be done fully experimentally. It is, however, beneficial to explore the results expected from theory.

Assuming that all parameters *a, b, c* and *d* are derived as described above, a generic expression for these four parameters can be made based on the following four conditions (referred to as equation 6):

The maximum pressure of pump scales linearly with the absolute pressure and with the square root of the absolute temperature

The maximum flow rate scales linearly with the absolute pressure and with the square root of the absolute temperature

The gradient $\frac{dQ_p}{d\Delta p_{cuff}}\left(\Delta p_{cuff} = 0\right)$ remains equal, hence *c* = constant (per pump)

The gradient $\left.\frac{dQ_p}{d\Delta p_{cuff}}(\Delta p_{max})\right|_{old} = \left.\frac{dQ_p}{d\Delta p_{cuff}}(\Delta p_{max})\right|_{new}$

The maximum pressure is given by equation (5). From requirements (6.1) and (6.2), we therefore have:

$$\Delta p_{max,new} = \Delta p_{max,ref} \frac{p_a(h)}{p_a(h_{ref})}\sqrt{\frac{T_{ref}}{T}} \tag{7.1}$$

$$Q_{p,max,new} = Q_{p,\ max,ref}\ \frac{p_a(h)}{p_a(h_{ref})}\sqrt{\frac{T_{ref}}{T}} \qquad (7.2)$$

Here, $p_a(h)$ is the relative atmospheric pressure as a function of the absolute height/altitude h (in meters), given by:

$$p(h) = 4.476 \cdot 10^{-9}\, h^2 - 1.1597 \cdot 10^{-4} h + 1 \qquad (8)$$

From requirement (6.4) and equation (1), the following can be found:

$$a_n = -\frac{2}{3}b_n\,\frac{1}{\Delta p_{max,new}} + a_{ref}\left(\frac{\Delta p_{max,ref}}{\Delta p_{max,new}}\right)^2 + \frac{2}{3}b_{ref}\,\frac{\Delta p_{max,ref}}{\Delta p_{max,new}^2} \qquad (9)$$

While from equation (7), another relation between $a_n$ and $b_n$ can be found:

$$\Delta p_{max,new}\left(a_n, b_n, c_{ref}, Q_{p,max,new}\right) =$$

$$\frac{p(h)}{p(h_{ref})}\sqrt{\frac{T_{ref}}{T}}\,\Delta p_{max,ref}\left(a_{ref}, b_{ref}, c_{ref}, Q_{p,max,ref}\right) \qquad (10)$$

Inserting equation (9) into equation (10), it is possible to numerically solve for $b_n$ and find $a_n$. This provides a generic description of the reference flow rate, i.e., $a_n$, $b_n$, $c_n$ and $Q_{max,n}$ are all known as a function of the reference $a$, $b$, c and $Q_{max}$ values and the absolute pressure and temperature. This method requires a numerical method to solve for $b_n$. Using the quadratic approach for the scaling of parameters $a_n$, $b_n$ and $Q_{max,n}$ is easier. First, $\Delta p_{max}$ can be calculated using equation (2):

$$\Delta p_{max} = -\frac{1}{2}\frac{a}{b}\left(1 - \sqrt{1 - \frac{4Q_{max}a}{b^2}}\right)$$

$$(11)$$

Scaling can then be done using equations (6) and (7). From (6.3), $b$ remains constant, while from (7), an immediate scaling of $Q_{max}$ can be obtained. From (6.4), or from the conditions for equation (7), for $\Delta p_{max}$, the following can be found:

$$a_{new} = \frac{p(h_{ref})}{p(h)}\sqrt{\frac{T}{T_{ref}}}\,a_{ref} \qquad (12)$$

This is easier than the calculation of the same parameter, i.e., for the $\Delta p^2$ term, for the cubic expression. Note that other functions of the temperature are also possible, i.e., $(T/T_{ref})^n$, where n can be any parameter between 0.1 and 10, and more preferably between 0.3 and 3. Further n could be a function of the PWM%.

The effect of voltage on the parameters $a$, $b$, c and $Q_{max}$ and $\Delta p_{max}$ can be determined experimentally, i.e., similarly as to the effect of PWM%. It has been observed that $Q_{max}$ scales linearly with voltage while $\Delta p_{max}$ approximately does not change with voltage. This can be seen in Fig. 4, which is a diagram illustrating the experimental results of the effect of voltage on a Q-H curve. As can be seen, this is a similar scaling as the PWM% (e.g., as seen in Fig. 3).

The scaling of the Q-H curve of the pump discussed here is for the reference flow rate, i.e. the flow rate at a reference pressure and temperature (1 bar and 21 °C). It is also possible to do a scaling of the volumetric flow rate instead of the reference flow rate. In this case, the volumetric flow rate is the flow rate at the actual total pressure, i.e. $p_a + \Delta p$, and the actual temperature. As the skilled person would appreciate, a similar procedure can be followed.

For example, the following equations can be used:

$$\Delta p_{max,new} = \Delta p_{max,ref} \frac{p_a(h)}{p_a(h_{ref})} \sqrt{\frac{T_{ref}}{T}}$$

(13.1)

$$Q_{vol,max,new} = Q_{vol,max,ref} \sqrt{\frac{T_{ref}}{T}}$$

(13.2)

$$c_n = c_{ref} \frac{p(h_{ref})}{p(h)} \frac{T}{T_{ref,pump}}$$

(13.3)

$$a_n = \frac{2}{3} b_n \frac{1}{\Delta p_{max,new}} + a_{ref} \left(\frac{\Delta p_{max,ref}}{\Delta p_{max,new}}\right)^2 \frac{p(h_{ref})}{p(h)} \frac{T}{T_{ref,pump}} -$$

$$\frac{2}{3} b_{ref} \frac{\Delta p_{max,ref}}{\Delta p_{max,new}^2} \frac{p(h_{ref})}{p(h)} \frac{T}{T_{ref,pump}}$$

(13.4)

While $b_n$ is calculated in a similar way, i.e., using:

$$\Delta p_{max,new}\left(a_n, b_n, c_{ref}, Q_{p,max,new}\right) =$$

$$\frac{p(h)}{p(h_{ref})} \sqrt{\frac{T_{ref}}{T}} \Delta p_{max,ref}\left(a_{ref}, b_{ref}, c_{ref}, Q_{p,max,ref}\right)$$

(13.5)

In a similar way as for the reference flow rate, $a_n$ is inserted in equation 13.5 and then $b_n$ can be solved and inserted back into equation 13.4 to find $a_n$. As the skilled person would appreciate, it may be advantageous to use the volumetric flow rate instead of the reference flow rate.

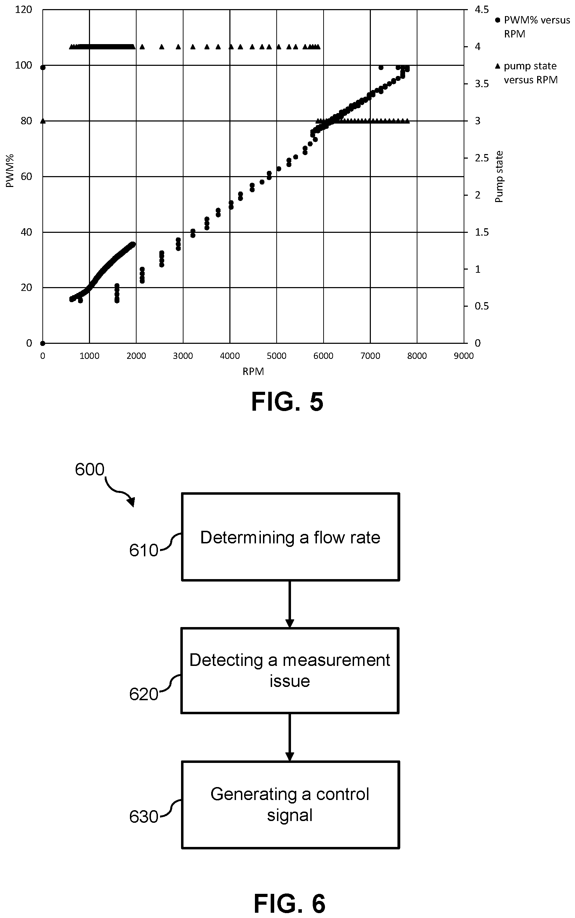

As mentioned, it is also possible to use RPM data of the pump instead of or in combination with the PWM%. Experimentally, there has been found to be a correlation of PWM% with RPM of the pump, where PWM% varies in accordance with the RPM dropping or increasing with time. This can be seen in Fig. 5, which is a diagram illustrating the experimental results of the correlation of PWM% with RPM and pump state.

It has also been found that when the RPM is dropping with time, i.e., just after starting, the PWM% appears to be lower than when the RPM is slowly increasing with time. This information can therefore be used to make a fitting based on RPM instead of PWM%. Furthermore, RPM and PWM% could be combined (or it could even be possible to include the history of the RPM or PWM% to improve the flow rate prediction). Further, the pump state could also be used to further improve prediction of the flow rate (i.e., accounting for what operational state the pump is in).

As the skilled person would understand, the reference total volume can be measured by integration of the reference flow rate over time.

It should also be noted that it is possible to not only use the average pump performance for the above, but also individual pump performance, where for example, the pump curve is corrected for data of each individual pump, e.g., max pressure and/or max flow, or the time to increase the pressure in a certain box of a given volume. The complete procedure would remain the same but the coefficients $a$, $b$, $c$ and $Q_{max}$ at a given reference temperature, ambient pressure, voltage and PWM% would vary per individual pump. This may provide a more accurate determination of flow rate.

Referring now to Fig. 6, there is depicted a flow diagram of a computer-implemented method 600 for determining a flow rate from a pump to an actuator of a blood pressure measurement system according to a proposed embodiment.

Step 610 is substantially the same as step 110 described in relation to method 100 of Fig. 1. Step 620 comprises detecting a measurement issue based on the determined flow rate. Based on the determined flow rate, it is possible to detect a wide range of issues with the blood pressure measurement system. For example, in this embodiment, the measurement issue comprises at least one of: a partial blockage of a regulating orifice; a full blockage of a regulating orifice; actuator leakage; a partial blockage of a tube coupling the actuator to a measuring device; and a full blockage of a tube coupling the actuator to a measuring device. These may be useful issues to detect as then said issues can be addressed and reliability of the blood pressure measurement system ensured, though as the skilled person would appreciate, in other embodiments, any suitable measurement issue can be detected (if it is possible to detect based on the determined flow rate).

A regulating orifice in the context of a blood pressure measurement system is a small opening or valve that controls the flow of fluid (usually air) into or out of the actuator (and can be situated in a measuring device of the blood pressure measurement system or in the actuator itself). This orifice is crucial for precisely regulating the pressure inside the actuator, allowing for controlled inflation and deflation of the blood pressure cuff (especially useful for shell cuffs). The size of the regulating orifice affects the rate at which pressure changes, enabling accurate measurements of blood pressure while minimizing fluctuations that could lead to erroneous readings. The regulating orifice ensures that the cuff applies consistent pressure on the arm, facilitating reliable blood pressure assessments. Based on the determined flow rate, it is thus possible to detect if said regulating orifice is partially or fully blocked.

In other words, the regulating orifice can be used to adjust the inflation rate of the actuator. For example, in a blood pressure measurement system, there can be four different cuff sizes, and without the regulating orifice, the smallest cuff size could be inflated too fast when a subject with a low blood pressure is measured. The inflation time would thus be too small to allow for sufficient breathing cycles, which are important for determining hemodynamic parameters.

Actuator leakage refers to unintended leaking of fluid (typically air) from the actuator, e.g., due to a loose connection or due to an unintended orifice.

The measuring device (e.g., a patient monitor) is the device which actually analyses measurements from the blood pressure measurement system and determines blood pressure values, and is connected to the actuator via a tube - full or partial blockages in this tube can thus be detected based on the determined flow rate.

In some embodiments, detecting the measurement issue (in step 620) can be further based on a subject parameter. This can ensure a more accurate detection of measurement issues. For example, a subject parameter can comprise at least one of: BMI; weight; height; age; gender; and arm circumference. Each of these can be useful subject parameters to facilitate more accurate detection of measuring issues.

Step 630 comprises generating a control signal based on the determined flow rate and the detected measurement issue. This thus facilitates automatically compensating for the detected measurement issue (e.g., to ensure a constant actuator pressure slope). For example, in this embodiment, the control signal comprises a control instruction for modifying an operating parameter of the pump. This is an effective way to compensate for the detected measurement issue. An operating parameter is any parameter which affects operation of the pump and, for example, in this embodiment, comprises at least one of: pulse width modulation percentage of the motor of the pump; revolutions per minute of the motor of the pump; voltage supplied to the motor of the pump; and user feedback. User feedback can be understood as anything which informs a user of some information, for example, controlling a display to output information for a user, turning on an LED which indicates a particular issue, or emitting a particular sound which indicates a particular issue, etc. The skilled person would appreciate that there are many ways in which user feedback can be provided.

In summary, in the state of the art, the flow rate as a function of time between a pump and an actuator of a blood pressure measurement system is not known (e.g., without the use of additional sensors). Knowledge of this flow rate, however, is useful as it allows, for example, detection of (partial or full blockage) of a regulating orifice that could be used especially in, for example, shell cuffs to allow for easier regulation of a low actuator inflation rate. Further, actuator leakage over time could be detected, as well as (partial or full) blockage/kinking of the tube connecting the measurement device (e.g., a patient monitor) with the actuator. Furthermore, knowledge of the (mass) flow rate can facilitate improved regulation of the actuator pressure slope.

In more detail, flow rate measurements/determinations can be used (in conjunction with a known orifice flow rate (e.g., obtained from a manufacturer)), for example, to detect partial or full blocking of a regulating orifice. For example, a partial blockage could be detected by determining a lower-than-expected flow rate (for a particular actuator pressure) - of course, this requires the assumption that the orifice was initially unblocked.

It is also possible to detect whether the regulating orifice is functioning correctly when subject data, such as BMI, weight, height, age, gender, and arm size (preferably, arm circumference at the middle of the length actuator) is incorporated. Based on the subject data, for example, it is possible to estimate the typical reference flow rate without the orifice - for example, it has been observed that flow rate is approximately constant without an orifice. Reference flow through a regulating orifice will strongly increase with actuator pressure, however, and hence a properly operating orifice can be easily detected as well as a partially or fully blocked orifice. A warning signal can then be given if a sufficiently blocked orifice is detected. For example, this can be done via audible signals, light indications, warning signals on displays or any other suitable communication means.

It should be noted that the (regulating) orifice can be positioned in the measurement device (e.g., the patient monitor), the tubing, or in the actuator (such that, for example, each cuff will have its own orifice). The latter option can be advantageous as then different cuffs can have different sized orifices, or no orifices at all, and depending on the situation, this might be desired.

In another example, an increase in reference flow rate overtime (e.g., of more than 0.05 l/min at 100-200 mm Hg actuator pressure) could indicate actuator leakage. Similarly to above, when sufficient actuator leakage is detected, a warning signal could be given. Furthermore, the tube connecting the measurement device with the actuator could be partially blocked. This would cause an increase in the resistance of the air flow to the actuator, but no blockage of the air flow to the orifice (if situated in the measurement device). When the tube is fully blocked, there will only be a flow to the orifice. This could thus be readily detected with knowledge of the flow rate of the orifice. If the flow rate delivered by the pump is (almost) the flow rate through the orifice and the tissue pressure is very slowly increasing, this is a clear sign of (partial) tube blockage. When a partial blockage of the supply tube is detected, a warning signal could be given.

It should also be noted that with knowledge of subject data (such as BMI, arm diameter, age, etc.), a better estimation can be made relating to the initial required volume to increase the actuator pressure. This can, in turn, be used to make better predictions of the required flow rate, and hence PWM% so that regulation can be improved once the reference flow rate is measured as a function of time.

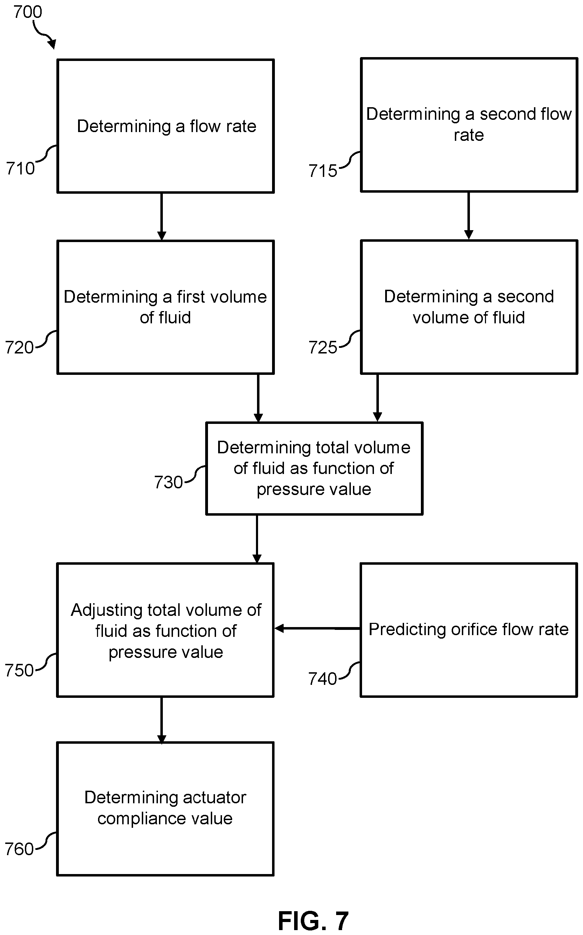

Referring now to Fig. 7, there is depicted a flow diagram of a computer-implemented method 700 for determining a flow rate from a pump to an actuator of a blood pressure measurement system according to a proposed embodiment.

Step 710 is substantially the same as step 110 described in relation to method 100 in Fig. 1.

Step 720 comprises determining a first volume of fluid (e.g., air) supplied to the actuator (from the pump) based on the determined flow rate. The skilled person would be readily aware of how this could be performed.

Step 715 is substantially the same as step 110 described in relation to method 100 in Fig. 1, however, using values from a different time point to those used in step 710. Step 715 thus comprises determining a second flow rate from the pump to the actuator based on a second pump operating parameter value and a second pressure value, wherein the second pump operating parameter value and the second pressure value describe a different time point to the time point which the pump operating parameter value and the pressure value describe.

Step 725 comprises determining a second volume of fluid supplied to the actuator based on the determined second flow rate in substantially the same was as in step 720, however, using different values.

Step 730 comprises determining a total volume of fluid supplied to the actuator as a function of pressure value based on the determined first and second volumes of fluid and the first and second pressure values. In other words, step 730 comprises determining a function relating the total volume of fluid supplied to the actuator and the pressure value, and this function is determined based on the first and second volumes of fluid and the first and second pressure values. The skilled person would be readily aware of the many ways in which this could be performed. The total volume of fluid supplied to the actuator as a function of pressure value is a particularly useful function to determine to assess operation of the blood pressure measurement system.

Of course, the skilled person would also be aware that many different actuator pressures, i.e., more than a two / a plurality, (and their corresponding volumes of fluid) would be desired to be used to determine the total volume of fluid supplied to the actuator as a function of pressure value such that said function can be accurately determined over an inflation cycle.

In this embodiment, the blood pressure measurement system comprises a regulating orifice (e.g., being substantially the same as the regulating orifice described in relation to step 620 and either in the measuring device, the tubing, or the actuator). Step 740 comprises predicting an orifice flow rate through the regulating orifice. The orifice flow rate can be predicted in any suitable way (e.g., theoretically based on the size/diameter of the orifice, the actuator pressure, temperature, and/or ambient pressure, or it could be experimentally measured, etc.).

Step 750 then comprises adjusting the determined total volume of fluid as function of pressure value based on the predicted orifice flow rate. This thus allows the total volume of fluid as a function of pressure value to be made more accurate. For example, a cuff can contain a regulating orifice which leaks air - however, by predicting the air flow through the regulating orifice (possible because the regulating orifices are made very accurately and reliably), the total volume of air as a function of pressure value can be made more accurate.

Of course, in other embodiments (including those where the total volume of fluid supplied to the actuator as a function of pressure value is not determined), the predicted orifice flow rate can instead/additionally be used to adjust the determined flow rate, thus making the flow rate more accurate (and also facilitating an effective determination of whether there is leakage present in the actuator).

It should be noted that in other embodiments, steps 740 and 750 can be skipped so that the method 700 essentially goes from step 730 straight to step 760.

Step 760 comprises determining an actuator compliance value based on the determined total volume of fluid as a function of pressure value and the first and second pressure values. The actuator compliance value is a useful parameter to derive for assessment of the operation of the blood pressure measurement system. The actuator compliance is, in essence, the gradient of the volumetric flow rate with respect to the actuator pressure, and thus the skilled person would readily appreciate how the actuator compliance value could be determined using the total volume of fluid as a function of pressure value and the first and second pressure values (and, in reality, likely many more pressure values).

In more detail, an extra benefit of the proposed flow rate determination is that the total volume of fluid (e.g., air) in the actuator can also be accurately determined. The total volume of air in the actuator as a function of the actuator pressure can then be used to calculate the actuator compliance, $C_a$. This can be expressed as:

$$C_a = \frac{\mathrm{d}V_a}{\mathrm{d}p_a} \qquad (14)$$

Typically, the actuator compliance is not constant, but rather is quickly reduced until it is almost constant. For example, for low actuator pressures, the compliance is typically large, whereas it drops for larger actuator pressures to become almost constant. It should be noted that the level of compliance varies with the size of the actuator.

A varying compliance is important as it can be used to correct the blood pressure envelope. Typically, the volume changes of the brachial artery of the subject results in a pressure change inside the actuator. Due to the non-constant compliance, the pressure pulses at low actuator pressure are often underestimated, resulting in an overestimation of the blood pressure values at low diastolic arterial pressure (DAP), mean arterial pressure (MAP) and systolic arterial pressure (SAP), which is not desired. This is due to the fact that the pressure change in the actuator resulting from a heart beat is given by:

$$\Delta p \approx \Delta V \cdot \frac{1}{C_a} \qquad (15)$$

Typically, the compliance is taken as constant, however, from the above, it can be understood that the compliance for low actuator pressure (e.g., 50-60 mm Hg) can be significantly larger than the values at greater actuator pressures (80-150 mm Hg). The pressure pulse height at low actuator pressure is therefore overestimated when assuming a constant compliance. This will therefore result in an overestimation of the DAP, MAP and SAP, and in cases where these values are in reality very low, this may cause health risks. More accurately determining the compliance (and correcting the pressure curves with the correct compliance) is therefore important to reduce errors, e.g., in determining the DAP, MAP and SAP.

It should also be noted that for oscillometric blood pressure measurement systems, a measurement of the total volume in the actuator during deflation of the actuator would be very useful. This is because it could be the case that the compliance as a function of actuator pressure during deflation is (slightly) different than during inflation. As the oscillometric blood pressure values are mainly measured during deflation, it is thus also important to know the compliance during deflation. This could be possible as, for example, the size of valves that are used in a control/measurement device are typically known, and hence the effective valve area can be known and the flow rate leaving the actuator through the valve(s) thus found from the measured pressure difference. By integrating the flow rate over time, the total volume leaving the actuator can be calculated and corrected for in the actuator, and in this way, it would also be possible to determine the compliance of the actuator during deflation.

In more detail, as mentioned above, compliance can be a very useful parameter to determine in order to correct measured blood pressure values (e.g., in an oscillometric non-invasive blood pressure meter). Normally, in an oscillometric blood pressure meter, the blood pressure is measured during deflation of the actuator - this is especially done to limit the influence of pump noise on the measurements. A method to determine compliance as a function of actuator pressure was described above - for during inflation of the actuator. However, although small, there can be some difference between the compliance during inflation and deflation. It would therefore be useful to determine the compliance during deflation of the actuator also.

In principle with the method described above, it is possible to determine the total volume during the inflation cycle and therefore also at the end of the inflation. For example, one or two valves inside the control box / measurement device may be opened through which air can flow out to the atmosphere. Similar to the regulating orifice, these valves typically have very tightly controlled opening areas, which are individually measured for each valve before they are installed. Therefore, with knowledge of the pressure difference as measured with, for example, a pressure sensor inside the control box / measurement device and the effective valve opening areas, the flow rate through each valve can be calculated by a fluid dynamic formula (e.g., the same as can be used to determine flow through the orifice).

The flow rate can thus be determined by the characterized effective cross-sectional area of the valve(s) and the measured pressure difference. The flow rate prediction through the valve can be made more accurate by taking into account ambient pressure and temperature. For example, for a valve, the volumetric flow rate through said valve can be described sufficiently accurately with:

$$Q_{vol} = \sqrt{\frac{\Delta pRT}{(p_a+\Delta p)k}}\frac{\pi}{4}d^2_{orifice} \qquad (16)$$

Equation (16) is a standard formula based on incompressible flow theory. Here, $R$ is the gas constant for air and equals 287 J/(kg K), $k$ is approximately equal to 1.1, and $\frac{\pi}{4}d^2_{orifice}$ are a resistance factor and an effective orifice area, respectively. They are determined by measuring the pressure profile to release the pressure in a well-defined volume. As the skilled person would understand, in other cases, more elaborate formulas where the effect of the compressibility of the gas is included can also be used. Also note that the volumetric flow rate is the flow rate at the pressure $p_a + \Delta p$ and absolute temperature $T$. This is a different flow rate compared to the reference flow rate where the flow rate is determined at reference temperature $T_{ref}$ and reference ambient pressure $p_{a, ref}$.

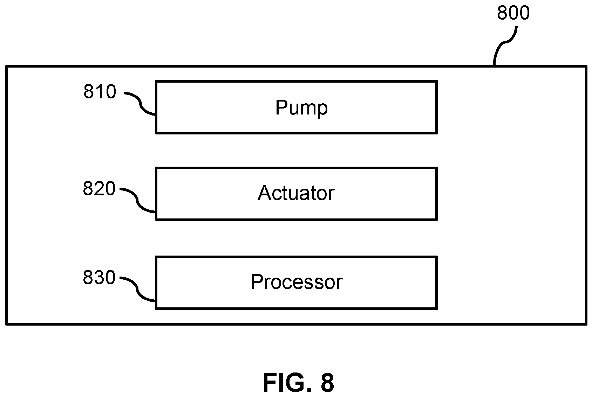

Referring now to Fig. 8, there is depicted a blood pressure measurement system 800. The system 800 comprises a pump 810, an actuator 820, and a processor 830. The pump 810 can comprise any suitable pump type, as has been discussed above. The actuator 820 likewise can comprise any suitable actuator type, such as a shell cuff actuator.

The processor 830 is configured to perform any herein-disclosed method (e.g., method 100, 600, or 700). For example, in this embodiment, the processor 830 is configured to determine a flow rate from the pump to the actuator based on a pump operating parameter value, describing operation of a motor of the pump, and a pressure value corresponding to a pressure inside the actuator.

In some embodiments, the blood pressure measurement system 800 can further comprise a pressure sensor for measuring the pressure value. For example, this could be placed within the actuator to directly measure the pressure inside the actuator or it could be placed somewhere suitable between the pump and the actuator such that it measures a pressure value corresponding/responsive to the pressure inside the actuator (i.e., and the measured value is a proxy for the pressure inside the actuator).

In some embodiments, the processor 830 can be the same processor that controls operation of the pump 810.

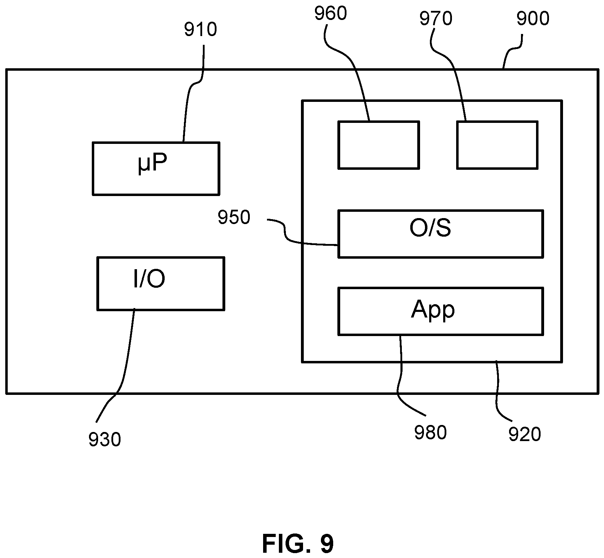

Fig. 9 illustrates an example of a computer 900 within which one or more parts of an embodiment may be employed. Various operations discussed above may utilize the capabilities of the computer 900. In this regard, it is to be understood that system functional blocks can run on a single computer or may be distributed over several computers and locations (e.g. connected via internet).

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 930 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1, 6 and 7, and the system of Fig. 8, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 9 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for determining a flow rate from a pump to an actuator of a blood pressure measurement system, the method comprising:
   determining a flow rate from the pump to the actuator (110) based on a pump operating parameter value, describing operation of a motor of the pump, and a pressure value corresponding to a pressure inside the actuator.

2. The computer-implemented method of claim 1, wherein the pump operating parameter value comprises at least one of: pulse width modulation percentage of the motor of the pump; and revolutions per minute of the motor of the pump.

3. The computer-implemented method of claim 1 or 2, wherein determining the flow rate from the pump to the actuator is further based on at least one of: voltage supplied to the motor of the pump; pump type; pump state of the pump; ambient pressure; altitude of the actuator; and ambient temperature.

4. The computer-implemented method any of claims 1 to 3, wherein the method further comprises: detecting a measurement issue (620) based on the determined flow rate.

5. The computer-implemented method of claim 4, wherein the measurement issue comprises at least one of: a partial blockage of a regulating orifice; a full blockage of a regulating orifice; actuator leakage; a partial blockage of a tube coupling the actuator to a measuring device; and a full blockage of a tube coupling the actuator to a measuring device.

6. The computer-implemented method of claim 4 or 5, wherein detecting the measurement issue is further based on a subject parameter, and preferably wherein a subject parameter comprises at least one of: BMI; weight; height; age; gender; and arm circumference.

7. The computer-implemented method of any of claims 4 to 6, wherein the method further comprises: generating a control signal (630) based on the determined flow rate and the detected measurement issue.

8. The computer-implemented method of claim 7, wherein the control signal comprises a control instruction for modifying an operating parameter of the pump, and preferably, wherein the operating parameter comprises at least one of: pulse width modulation percentage of the motor of the pump; revolutions per minute of the motor of the pump; voltage supplied to the motor of the pump; and user feedback.

9. The computer-implemented method of any of claims 1 to 8, wherein the method further comprises:

   determining a first volume of fluid supplied to the actuator (720) based on the determined flow rate;
   determining a second flow rate from the pump to the actuator (715) based on a second pump operating parameter value and a second pressure value, wherein the second pump operating parameter value and the second pressure value describe a different time point to the pump operating parameter value and the pressure value;
   determining a second volume of fluid supplied to the actuator (725) based on the determined second flow rate;

and

determining a total volume of fluid supplied to the actuator as a function of pressure value (730) based on the determined first and second volumes of fluid and the first and second pressure values.

10. The computer-implemented method of claim 9, wherein the blood pressure measurement system comprises a regulating orifice, and wherein the method further comprises:

predicting an orifice flow rate through the regulating orifice (740); and
adjusting the determined flow rate and/or the determined total volume of fluid as a function of pressure value (750) based on the predicted orifice flow rate.

11. The computer-implemented method of claim 9 or 10, further comprising determining an actuator compliance value based on the determined total volume of fluid as a function of pressure value and the first and second pressure values.

12. The computer-implemented method of any of claims 1 to 11, wherein the blood pressure measurement system is a non-invasive blood pressure measurement system.

13. The computer-implemented method of any of claims 1 to 12, wherein the actuator comprises a shell cuff actuator.

14. A computer program comprising code for implementing the method of any preceding claim when said program is run on a processor.

15. A blood pressure measurement system (800) comprising:

a pump (810);
an actuator (820); and
a processor (830) configured to:
determine a flow rate from the pump to the actuator based on a pump operating parameter value, describing operation of a motor of the pump, and a pressure value corresponding to a pressure inside the actuator.

**FIG. 1**

**FIG. 2A**

**FIG. 2B**

FIG. 2C

FIG. 2D

FIG. 2E

**FIG. 3**

**FIG. 4**

FIG. 5

FIG. 6

700

710 — Determining a flow rate

720 — Determining a first volume of fluid

715 — Determining a second flow rate

725 — Determining a second volume of fluid

730 — Determining total volume of fluid as function of pressure value

750 — Adjusting total volume of fluid as function of pressure value

740 — Predicting orifice flow rate

760 — Determining actuator compliance value

**FIG. 7**

800

| 810 | Pump |
|---|---|
| 820 | Actuator |
| 830 | Processor |

**FIG. 8**

**FIG. 9**

Europäisches
Patentamt

European
Patent Office

Office européen
des brevets

## EUROPEAN SEARCH REPORT

**Application Number**

EP 24 21 1955

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/338085 A1 (KUENEN MAARTEN PETRUS JOSEPH [NL]) 4 November 2021 (2021-11-04) * paragraph [0055] * ----- | 1-3, 12-15 | INV. A61B5/0225 |
| X | US 2018/235488 A1 (AELEN PAUL [NL]) 23 August 2018 (2018-08-23) * paragraphs [0032], [0145] * ----- | 1-3, 12-15 | |
| X | US 2014/257116 A1 (KOBAYASHI TATSUYA [JP] ET AL) 11 September 2014 (2014-09-11) * paragraphs [0009], [0066] * ----- | 1-3, 12-15 | |
| A | CN 115 137 328 A (ZOLL MEDICAL CORP) 4 October 2022 (2022-10-04) * paragraph [0046] * ----- | 1-3, 12-15 | |

**TECHNICAL FIELDS SEARCHED (IPC)**

A61B

The present search report has been drawn up for all claims

| Place of search | Date of completion of the search | Examiner |
|---|---|---|
| The Hague | 22 April 2025 | Clevorn, Jens |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...........................................................................
& : member of the same patent family, corresponding
document

EPO FORM 1503 03.82 (P04C01)

Europäisches
Patentamt

European
Patent Office

Office européen
des brevets

**Application Number**

EP 24 21 1955

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-3, 12-15

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**
**SHEET B**

Application Number

**EP 24 21 1955**

The Search Division considers that the present European patent application does not comply with the requirements of unity of invention and relates to several inventions or groups of inventions, namely:

      1. claims: 1-3, 12-15

           additional features of claim 2
                    ---

      2. claims: 4-8

           additional feature of claim 4
                    ---

      3. claims: 9-11

           additional features of claim 9
                    ---

EP 4 740 852 A1

## ANNEX TO THE EUROPEAN SEARCH REPORT
## ON EUROPEAN PATENT APPLICATION NO.

EP 24 21 1955

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2025

| Patent document cited in search report | | Publication date | Patent family member(s) | | Publication date |
|---|---|---|---|---|---|
| US 2021338085 | A1 | 04-11-2021 | CN | 112739256 A | 30-04-2021 |
| | | | EP | 3628217 A1 | 01-04-2020 |
| | | | EP | 3856017 A2 | 04-08-2021 |
| | | | JP | 6998496 B2 | 18-01-2022 |
| | | | JP | 2021529642 A | 04-11-2021 |
| | | | US | 2021338085 A1 | 04-11-2021 |
| | | | WO | 2020064581 A2 | 02-04-2020 |
| US 2018235488 | A1 | 23-08-2018 | CN | 107920765 A | 17-04-2018 |
| | | | EP | 3344130 A1 | 11-07-2018 |
| | | | JP | 6849663 B2 | 24-03-2021 |
| | | | JP | 2018529417 A | 11-10-2018 |
| | | | US | 2018235488 A1 | 23-08-2018 |
| | | | WO | 2017037272 A1 | 09-03-2017 |
| US 2014257116 | A1 | 11-09-2014 | CN | 103889320 A | 25-06-2014 |
| | | | DE | 112012004471 T5 | 10-07-2014 |
| | | | JP | 5811766 B2 | 11-11-2015 |
| | | | JP | 2013090825 A | 16-05-2013 |
| | | | US | 2014257116 A1 | 11-09-2014 |
| | | | WO | 2013061779 A1 | 02-05-2013 |
| CN 115137328 | A | 04-10-2022 | NONE | | |

EPO FORM P0459

For more details about this annex : see Official Journal of the European Patent Office, No. 12/82

27